# EUROPEAN PATENT SPECIFICATION

(11) **EP 0 846 689 B1**
(45) Date of publication and mention of the grant of the patent: **14.01.2004**
(21) Application number: 97309761.1
(22) Date of filing: 03.12.1997
(51) Int. Cl.: C07D 235/08, C07D 235/10, C07D 235/12, C07D 235/14, C07D 235/16, C07D 401/06, C07D 413/06, A61K 31/415, A61K 31/42, A61K 31/44

(54) **Benzimidazole compounds**
Benzimidazol-Verbindungen
Composés de benzimidazole

(30) Priority: 09.12.1996 WO PCT/IB96/01395
(43) Date of publication of application: 10.06.1998
(73) Proprietor: PFIZER INC., New York, N.Y. 10017 (US)
(72) Inventor: Mano, Takashi, Handa-shi, Aichi-ken 475 (JP); Okumura, Yoshiyuki, Chita-shi, Aichi-ken 478 (JP); Stevens, Rodney W., Handa-shi, Aichi-ken 475 (JP)
(74) Representative: Ruddock, Keith Stephen

(56) References cited:
- EP-A- 0 419 210
- EP-A- 0 608 548
- DE-A- 1 962 353
- US-A- 3 586 670
- CHEMICAL ABSTRACTS, vol. 82, no. 9, 3 March 1975 Columbus, Ohio, US; abstract no. 57696d, page 574; XP002059542 & JP 49 081 369 A (TOBISHI PHARMACEUTICAL CO., LTD.) 6 August 1974
- PATENT ABSTRACTS OF JAPAN vol. 8, no. 183 (P-296), 23 August 1984 & JP 59 075257 A (RICOH KK), 27 April 1984,

## Description

### Technical Field

This invention relates to novel benzimidazole cyclooxygenase inhibitors. The compounds of this invention inhibit the biosynthesis of prostaglandins by intervention of the action of the enzyme cyclooxygenase on arachidonic acid, and are therefore useful in the treatment or alleviation of inflammation and other inflammation associated disorders in mammals. This invention also relates to pharmaceutical compositions comprising such compounds.

### Background Art

Nonsteroidal antiinflammatory drugs (NSAIDs) are widely used in treating pain and the signs and symptoms of arthritis because of their analgesic and antiinflammatory activity. It is accepted that common NSAIDs work by blocking the activity of cyclooxygenase (COX), also known as prostaglandin G/H synthase (PGHS), the enzyme that converts arachidonic acid into prostanoids. Prostaglandins, especially prostaglandin E₂ (PGE₂), which is the predominant eicosanoid detected in inflammation conditions, are mediators of pain, fever and other symptoms associated with inflammation. Inhibition of the biosynthesis of prostaglandins has been a therapeutic target of antiinflammatory drug discovery. However, use of conventional NSAIDs is limited due to adverse side effects, notably gastrointestinal and renal toxicities.

Recently, two forms of COX were identified, a constitutive isoform (COX-1) and an inducible isoform (COX-2), of which expression is upregulated at sites of inflammation (Vane, J. R.; Mitchell, J. A.; Appleton, I.; Tomlinson, A.; Bishop-Bailey, D.; Croxtoll, J.;Willoughby, D. A. *Proc. Natil*. *Acad. Sci. USA,* **1994**, *91,* 2046). COX-1 is thought to play a physiological role and to be responsible for gastrointestinal and renal protection. On the other hand, COX-2 appears to play a pathological role and to be the predominant isoform present in inflammation conditions. A pathological role for prostaglandins has been implicated in a number of human disease states including rheumatoid and osteoarthritis, pyrexia, asthma, bone resorption, cardiovascular diseases, nephrotoxicity, atherosclerosis, hypotension, shock, pain, cancer, and Alzheimer disease. The NSAIDs currently on market inhibit both isoforms of COX with little variation for selectivity, explaining their beneficial (inhibition of COX-2) and deleterious effects (inhibition of COX-1). It is possible that a selective inhibitor of COX-2 may eliminate the side effects associated with COX-1 inhibition while providing anti-inflammatory effects.

A variety of benzimidazole compounds are known and are disclosed in several patent applications. Specifically, Japanese Kokai (laid-open) Publication Number S49-81369 discloses 1-benzyl-benzimidazole compounds as anti-inflammatory agents. Japanese Kokai (laid-open) Publication Number S59-75257 and H06-194780 disclose a variety of benzimidazole compounds as electrophotographic materials.

### Brief Disclosure of the Invention

The present invention provides a compound of the following formula: 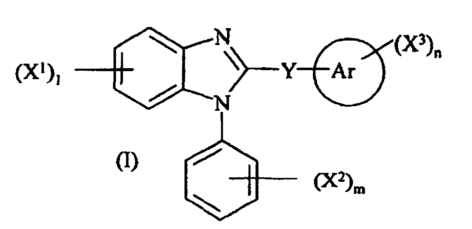 and the pharmaceutically acceptable salts thereof wherein
**Ar** is phenyl, C₃₋₈ cycloalkyl or heteroaryl which is connected to Y through a carbon atom, the heteroaryl being selected from pyridyl and oxazolyl;
**X**^{**1**} is H, halo, amino, cyano or nitro;
**X**^{**2**} and **X**^{**3**} are independently halo;
**Y** is ―CR¹=CR²- wherein R¹ and R² are independently H or methyl;
1 is 0 or 1; and
m and n are independently 0 or 1;
with the proviso that when Ar is phenyl; and l, m and n are 0, Y is not -CH=CH-

The benzimidazole compounds of the present invention exhibit inhibition of COX activity. Preferably compounds of this invention exhibit inhibitory activity against COX-2, with more preferable compounds having COX-2 selectivity.

Accordingly, the present invention also provides a pharmaceutical composition, useful for the treatment of a medical condition in which prostaglandins are implicated as pathogens, which comprises a compound of the formula (I): wherein Ar, Y, X¹, X², X³, l, m and n are as defined above, and the pharmaceutically acceptable salts thereof.

Further, the present invention provides a method for the treatment of a medical condition in which prostaglandins are implicated as pathogens, in a mammalian subject, which comprises administering to said subject a therapeutically effective amount of said pharmaceutical composition.
The medical conditions in which prostaglandins are implicated as pathogens, include the relief of pain, fever and inflammation of a variety of conditions including rheumatic fever, symptoms associated with influenza or other viral infections, common cold, low back and neck pain, dysmenorrhea, headache, toothache, sprains and strains, myositis, neuralgia, synovitis, arthritis including rheumatoid arthritis, degenerative joint disease (osteoarthritis) gout and ankylosing spondylitis, bursitis, burns, injuries following surgical and dental procedures.

The compounds and pharmaceutical composition of this invention may inhibit cellular neoplastic transformations and metastic tumor growth and thus may be used in the treatment of cancer. The compounds and pharmaceutical composition of this invention were used in the treatment and/or prevention of cyclooxygenase-mediated proliferation disorders such as which occur in diabetic retinopathy and tumor angiogenesis.

The compounds and pharmaceutical composition of this invention may be of use in the treatment of dysmenorrhea, premature labor, asthma and eosinophil related disorders, use in the treatment of Alzheimer's disease and for the treatment of bone loss (treatment of osteoarthritis) by their ability to inhibit prostaniod-induced smooth muscle contraction by preventing the synthesis of contractile prostanoids.

Furthermore, such compounds and pharmaceutical compositions which show specificity for COX-2 over COX-1, will prove useful as an alternative to conventional NSAIDs particularly where such NSAIDs may be contra-indicated such as in patients with peptic ulcers, gastritis, regional enterotis, ulcerative colitis, diverticulitis or with a redurrent history of GI lesions, GI bleeding, coagulation disorders including anemia such as hypoprothrombinemia, haemophilia or other bleeding problems; kidney disease; prior to surgery of taking of anticoagulants.

### Detailed Disclosure of the Invention

As used herein, "halo" is fluoro, chloro, bromo or iodo.

Preferred compounds of this invention are those of the formula (I) wherein **Ar** is phenyl or C₃₋₈ ccycloalkkyl; **X**^{**2**} and **X**^{**3**} are independently halo, **X**^{**1**} is H, halo, amino, cyano or nitro, which is attached to 5- or 6-ring position of the benzimidazole ring system; and **Y** is -CR¹ = CR²-, wherein **R**^{**1**} and **R**^{**2**} are independently H or methyl.

Further preferred compounds of this invention are those of the formula (I) wherein **Ar** is phenyl, cyclopentyl or cyclohexyl; **X**^{**2**} and **X**^{**3**} are fluoro; **X**^{**1**} is H, fluoro, chloro, bromo, amino, cyano or nitro, which is attached to the 5-ring position of the benzimidazole ring system; and **Y** is -CH=CH-.

Preferred individual compounds of this invention are:
(*E*)-2-(4-fluorostyryl)-1-phenyl-1*H*-benzimidazole;
(*E*)-2-(2-fluorostyryl)-1-phenyl-1*H*-benzimidazole hydrochloride;
(*E*)-5-nitro-1-phenyl-2-styryl-1*H*-benzimidazole;
(*E*)-5-amino-1-phenyl-2-styryl-1*H*-benzimidazole dihydrochloride;
(*E*)-5-bromo-1-phenyl-2-styryl-1*H*-benzimidazole;
(*E*)-5-cyano-1-pheny-2-styryl-1*H*-benzimidazole;
(*E*)-2-(2-cyclohexylvinyl)-1-phenyl-1*H*-benzimidazole;
(*E*)-2-(2-cyclopentylvinyl)-1-phenyl-1*H*-benzimidazole hydrochloride; and
(*E*)-1-phenyl-2-[2-(2-pyridyl)vinyl]-1*H*-benzimidazole.

Particularly preferred individual compounds are:
(*E*)-5-nitro-1-phenyl-2-styryl-1*H*-benzimidazole;
(*E*)-5-cyano-1-phenyl-2-styryl-1*H*-benzimidazole; and
(*E*)-2-(2-cyclopentylvinyl)-1-phenyl-1*H*-benzimidazole hydrochloride.

Preferred pharmaceutical compositions of this invention comprise those compounds of the formula (I), wherein **Ar** is phenyl, C₃₋₈ cycloalkyl or heteroaryl selected from pyridyl and oxazolyl; **X**^{**2**} and **X**^{**3**} are independently halo; **X**^{**1**} is H, halo, amino, cyano or nitro; 1 is 0 or 1; and m and n are independently 0 or 1.

More preferred pharmaceutical compositions of this invention comprise those compounds of the formula (I), wherein **Ar** is phenyl or C₃₋₈ cycloalkyl; **X**^{**2**} and **X**^{**3**} are independently halo, **X**^{**1**} is H, halo, amino, cyano or nitro, which is attached to 5- or 6-ring position of the benzimidazole ring system; and **Y** is -CR¹=CR²-, wherein **R**^{**1**} and **R**^{**2**} are independently H or methyl.

Furthermore, preferred pharmaceutical compositions of this invention comprise those compounds of the formula (I), wherein **Ar** is phenyl, cyclopentyl or cyclohexyl; **X**^{**2**} and **X**^{**3**} are fluoro; **X**^{**1**} is H, fluoro, chloro, bromo, amino, cyano or nitro, which is attached to the 5-ring position of the benzimidazole ring system; and **Y** is -CH=CH-.

Preferred individual compounds to be contained in the pharmaceutical compositions are:
(*E*)-1-phenyl-2-styryl-1*H*-benzimidazole;
(*E*)-2-(4-fluorostyryl)-1-phenyl-1*H*-benzimidazole;
(*E*)-2-(2-fluorostyryl)-1-phenyl-1*H*-benzimidazole hydrochloride;
(*E*)-5-nitro-1-phenyl-2-styryl-1*H*-benzimidazole;
(*E*)-5-amino-1-phenyl-2-styryl-1*H*-benzimidazole dihydrochloride;
(*E*)-5-bromo-1-phenyl-2-styryl-1*H*-benzimidazole;
(*E*)-5-cyano-1-phenyl-2-styryl-1*H*-benzimidazole;
(*E*)-2-(2-cyclohexylvinyl)-1-phenyl-1*H*-benzimidazole;
(*E*)-2-(2-cyclopentylvinyl)-1-phenyl-1*H*-benzimidazole hydrochloride; and
(*E*)-1-phenyl-2-[2-(2-pyridyl)vinyl]-1*H*-benzimidazole.

Particularly preferred individual compounds to be contained in the pharmaceutical composition of this invention are:
(*E*)-1-phenyl-2-styryl-1*H*-benzimidazole;
(*E*)-5-nitro-1-phenyl-2-styryl-1*H*-benzimidazole;
(*E*)-5-cyano-1-phenyl-2-styryl-1*H*-benzimidazole; and
(*E*)-2-(2-cyclopentylvinyl)-1-phenyl-1*H*-benzimidazole hydrochloride.

The compounds of the formula (I) of this invention can be administered via either the oral, parenteral or topical routes to mammals. In general, these compounds are most desirably administered to humans in doses ranging from 0.01 mg to 100 mg per kg of body weight per day, although variations will necessarily occur depending upon the weight, sex and condition of the subject being treated, the disease state being treated and the particular route of administration chosen. However, a dosage level that is in the range of from 0.1 mg to 10 mg per kg of body weight per day, single or divided dosage is most desirably employed in humans for the treatment of abovementioned diseases.

The compounds of the present invention may be administered alone or in combination with pharmaceutically acceptable carriers or diluents by either of the above routes previously indicated, and such administration can be carried out in single or multiple doses. More particularly, the novel therapeutic agents of the invention can be administered in a wide variety of different dosage forms, i.e., they may be combined with various pharmaceutically acceptable inert carriers in the form of tablets, capsules, lozenges, trochees, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various nontoxic organic solvents, etc. Moreover, oral pharmaceutical compositions can be suitably sweetened and/or flavored. In general, the therapeutically-effective compounds of this invention are present in such dosage forms at concentration levels ranging 5% to 70% by weight, preferably 10% to 50% by weight.

For oral administration, tablets containing various excipients such as microcrystalline cellulose, sodium citrate, calcium carbonate, dipotassium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatine capsules; preferred materials in this connection also include lactose or milk sugar as well as high molecular weight polyethylene grycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH>8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

### General Synthesis

A compound of formula (I) may be prepared by any synthetic procedure applicable to structure-related compounds known to those skilled in the art. The following representative examples as described in Schemes I-VII are illustrative of the invention in which, unless otherwise stated, Ar, X¹, X², X³ and Y are as defined herein before. For the synthesis of compounds of related-structure to compounds of the present invention, see "Benzimidazoles and Congeneric Tricyclic Compounds" in *Heterocyclic Compounds,* Vol. **40**, Preson, P. N. Ed., John Wiley & Sons, NY, **1981**.

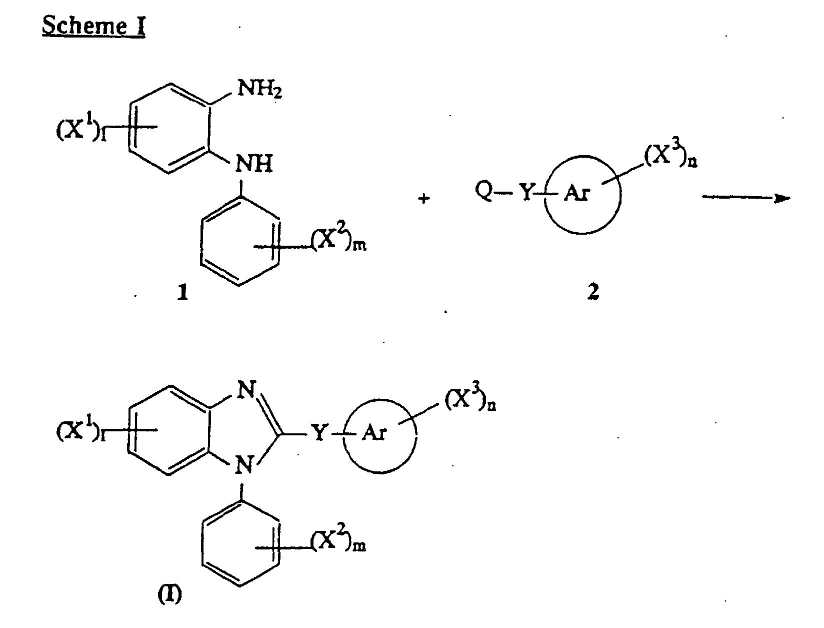

For example, the compound of formula (I) may be prepared according to the reaction outlined in Scheme I. In the instant example, a phenylenediamine compound of formula 1 is reacted with a compound of formula 2 wherein the group Q is a residue of a carboxylic acid, carboxylic acid ester, carboxamide, carboxylic acid anhydride, carboxylic acid chloride, orthoester, imino ether, a carbaldehyde or the like. The reaction may be conducted in the presence or absence of a reaction inert solvent. Preferred reaction inert solvents include benzene, toluene, xylene, pyridine, 1,2-dichloroethane, o-dichlorobenzene, nitrobenzene and dichloromethane. Preferably, the reaction is conducted in the presence of a promoter such as hydrochloric acid, polyphosphoric acid, phosphorous pentoxide, phosphorous oxychloride, polyphosphoric acid ethyl ether, polyphosphoric acid trimethylsilyl ether, *p*-toluenesulfonic acid, zinc (II) chloride or the like. When a compound of formula 2 is carboxaldehyde, the reaction may be conducted in the presence of an oxidant such as cupric acetate, chloranil, or the like. Reaction temperatures are preferably in the range of -40 °C to 250 °C, usually in the range of 20 °C to 200 °C, but if necessary, lower or higher temperature can be employed. Reaction time may vary, in general, from 5 minutes to 6 days, preferably from 20 minutes to 1 day. Alternatively, the reaction may be conducted in a sealed tube or an autoclave at medium (1-10 kg/cm²) to high pressure (20-200 kg/cm²) to accelerate it, preferably in the range of 2 to 150 kg/cm².

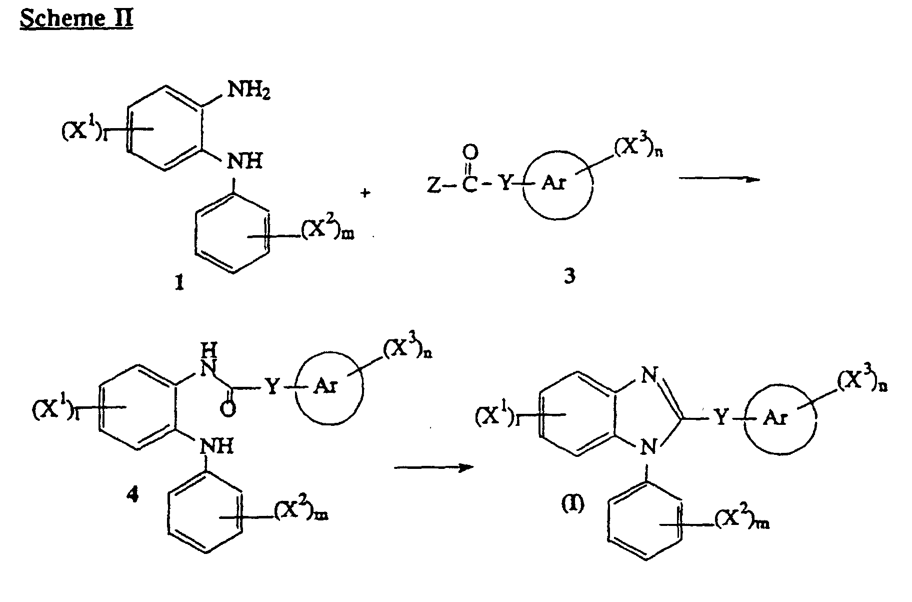

Alternatively, the compounds of formula (I) may be prepared by a two step procedure from phenylenediamine compounds of formula 1 via the (*N*-acylamino)phenylamine compounds of formula 4 as shown in Scheme II. In the first step, a phenylenediamine compound of formula 1 is reacted with a compound of formula 3, wherein Z is selected from halo, -OH, -OR (R is C₁₋₄ alkyl), -NH₂, and -OC(O)Y-Ar-(X³)ₙ, by conventional methods known to those skilled the art to form amides of formula 4. For example, when a compound of formula 3 is carboxylic acid (i.e, Z is OH), the reaction is preferably conducted in the presence of a coupling reagent such as 1-(dimethylaminopropyl)-3-ethylcarbodiimide (WSC), *N,N'*-dicyclohexylcarbodiimidazole (DCC), carbonyldiimidazole, cyanophosphonic acid diethyl ester or the like. Preferred reaction-inert solvents include acetone, acetonitrile, dichloromethane, *N,N*-dimethylformamide, *N*,*N*-dimethylacetamide, dimethylsulfoxide, dioxane, tetrahydrofuran and pyridine.

In next step, the compounds of formula (I) are provided by cyclization of the compounds of formula 4. The reaction may be conducted in the presence or absence of a reaction inert solvent. Preferred reaction inert solvents include benzene, toluene, xylene, pyridine, 1,2-dichloroethane, *o*-dichlorobenzene, nitrobenzene, dichloromethane and ethanol. Preferably, the reaction is conducted in the presence of a promoter such as of hydrochloric acid, polyphosphoric acid, phosphorous pentoxide, phosphorous oxychloride, polyphosphoric acid ethyl ether, polyphosphoric acid trimethylsilyl ether, thionyl chloride, *p*-toluenesulfonic acid, or the like. Alternatively, the cyclization reaction may be performed under Mitsunobu-type reaction conditions, for example, in the presence of triphenylphosphine and diethyl azodicarboxylate. Reaction temperatures are preferably in the range of -40 °C to 250 °C, usually in the range of 20 °C to 200 °C, but if necessary, lower or higher temperature can be employed. Reaction time may vary, in general, from 5 minutes to 6 days, preferably from 20 minutes to 1 day.

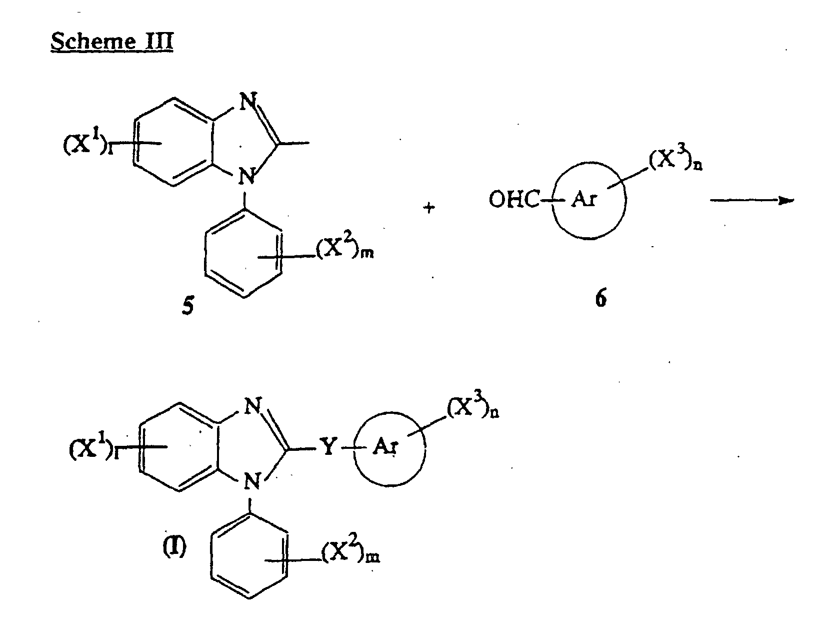

In another embodiment, the compounds of formula (I) wherein Y is C(H)=C(H) may be prepared as shown in Scheme III. Thus, 2-methylbenzimidazole compounds of formula 5 are reacted with aldehydes of formula 6 in the presence or absence of base. When the said reaction is conducted in the absence of base, the reaction is preferably performed in a sealed tube or an autoclave at medium (1-10 kg/cm²) to high pressure (20-200 kg/cm²), preferably in the range of 2 to 150 kg/cm². The reaction may be conducted in the presence or absence of a reaction inert solvent. Preferred reaction inert solvents include benzene, toluene, xylene, chlorobenzene, nitrobenzene, acetic acid, acetic anhydride and the like. Reaction temperatures are generally in the range of -100 °C to 250 °C, preferably in the range of 20 °C to 200 °C, but if necessary, lower or higher temperature can be employed. Reaction time may vary, in general, from 5 minutes to a day, preferably from 20 minutes to 5 hours, however shorter or longer reaction times if necessary can be employed. When the said reaction is conducted in the presence of base, reaction temperatures are generally in the range of -100 °C to 250 °C, preferably in the range of -80 °C to 20 °C, but if necessary, lower or higher temperature can be employed. Preferred reaction inert solvents include THF, benzene, toluene and xylenes. Reaction time may vary, in general, from 5 minutes to one day, preferably from 20 minutes to 5 hours, however shorter or longer reaction time, if necessary, can be employed. Preferred bases include, for example, an alkali or alkaline earth metal hydroxide, alkoxide, carbonate or hydride, such as sodium hydroxide, potassium hydroxide, sodium methoxide, sodium ethoxide, potassium *tert*-butoxide, sodium carbonate, potassium carbonate, sodium hydride or potassium hydride; an amine such as triethylamine, diisopropylamine, diisopropylethylamine, piperidine or dimethylaminopyridine; and an alkyl lithium such as *n*-butyl lithium, *sec*-butyl lithium, *tert*-butyl lithium, methyl lithium or lithium diisopropylamide.

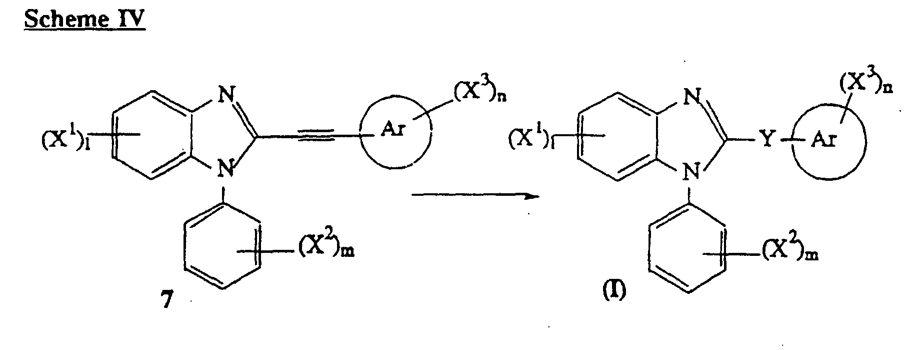

In another embodiment, the compounds of formula (I) wherein Y is C(H)=C(H) may be prepared by partial hydrogenation of a compound of formula (I) wherein Y is C-≡-C as depicted in Scheme IV. Preferred catalysts include, for example, nickel-based catalysts such as P-2 nickel and nickel boride (Choi,J; Yoon, N. M. *Tetrahedron Lett.,* **1996**, *37,* 1057) and palladium-based catalysts such as Lindlar catalyst and Pd/W. Preferred reaction-inert solvents include, for example, water, methanol, ethanol, acetone, acetonitrile, ethyl acetate, dichloromethane, dioxane, tetrahydrofuran, diethyl ether and diisopropyl ether. Reaction temperatures are preferably in the range of -40 °C to 200 °C, usually in the range of 20 °C to reflux temperature of solvent, but if necessary, lower or higher temperature can be employed. Reaction time is in general from 5 minutes to 6 days, preferably from 100 minutes to 5 days.

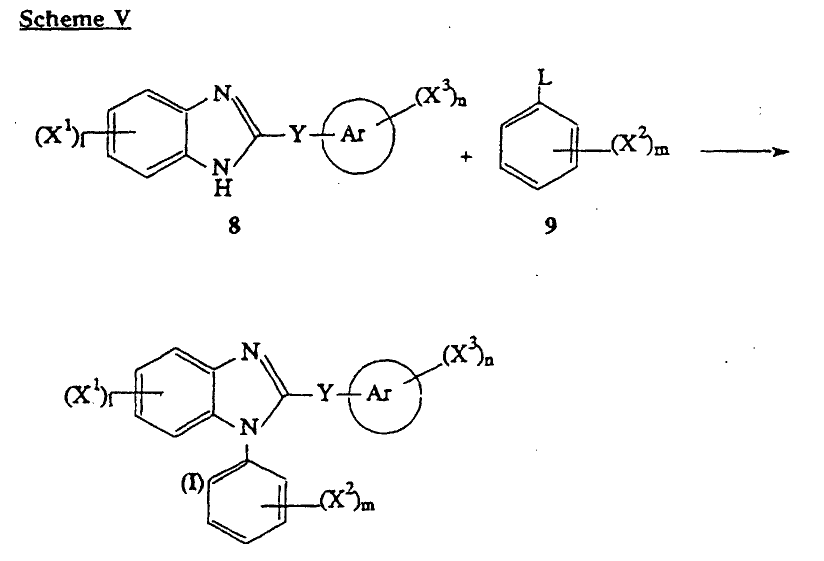

The compounds of formula (I) may also be prepared by reacting a compound of formula 8 with a compound of formula 9 according to the procedure outlined in Scheme V. In Scheme V, the compound of formula 8 may be synthesized by any of the methods described in Schemes I to IV herein before. The group L of the compounds of formula 9 is selected from suitable displaceable groups, for example, halo such as fluoro, chloro, bromo or iodo, and sulfonyloxy such as trifluoromethanesulfonyloxy, methanesulfonyloxy or *p*-toluenesulfonyloxy, all readily accessible by conventional methods known to those skilled in the art. Preferably, the instant reaction is conducted in the presence of a suitable base, for example, an alkali or alkaline earth metal hydroxide, alkoxide, carbonate, or hydride, such as sodium hydroxide, potassium hydroxide, sodium methoxide, sodium ethoxide, potassium tert-butoxide, sodium carbonate, potassium carbonate, sodium hydride or potassium hydride, or in the presence of an organic base an amine such as triethylamine, diisopropylethylamine diisopropylamine, or dimethylaminopyridine. Preferred reaction-inert solvents include acetone, acetonitrile, dichloromethane, *N,N*-dimethylformamide, *N,N*-dimethylacetamide, dimethylsulfoxide, dioxane, tetrahydrofuran and pyridine. Reaction temperatures are preferably in the range of -40 °C to 200 °C, usually in the range of 20 °C to reflux temperature of solvent, but if necessary, lower or higher temperature can be employed. Reaction time is in general from 5 minutes to 6 days, preferably from 30 minutes to 5 days. Conveniently, the reaction may be conducted in the presence of a suitable catalyst, for example, tetrakis(triphenylphosphine)-palladium, bis(triphenylphosphine)palladium (II) chloride, copper (0), cuprous chloride, cuprous oxide, cuprous iodide, cuprous bromide or cuprous chloride.

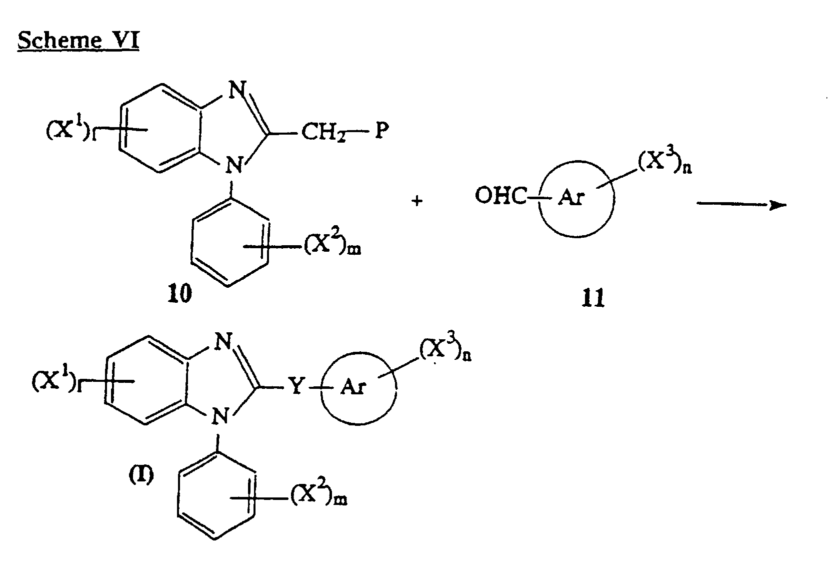

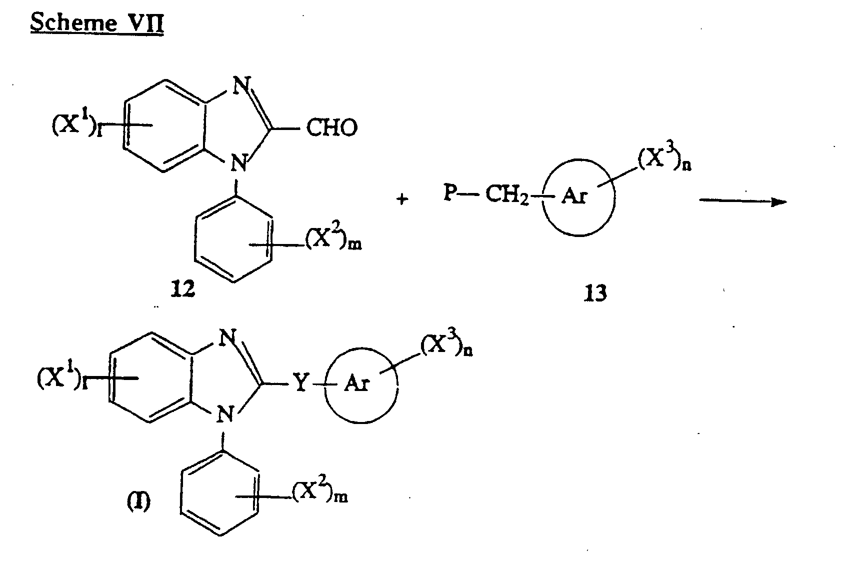

Alternatively, the compounds of formula (I) wherein Y is C(H)=C(H) may be prepared by the reaction of a suitable aldehyde with a suitable phosphonium (Maryanoff, B. E.; Reitz, A. B. *Chem. Rev.* **1989**, *89*, 863) or a dialkyl phosphonate salt (Seguineau, ; Villieras, *Tetrahedron Lett.* **1988**, *29*, 477) as shown in Schemes VI and VII, wherein P is a suitable phoshonium or dialkyl phosphonate salt. For appropriate references, see DE1939809A.

The starting material of formulae 1, 2, 3, 4, 5, 6, 9, 10, 11, 12 and 13 may be obtained by conventional procedures known to those skilled in the art. The preparation of such starting materials is described within the accompanying non-limiting examples which are provided for the purpose of illustration only. Alternatively, requisite starting materials may be obtained by analogous procedures, or modifications thereof described hereinafter.

The products which are addressed in the aforementioned general synthesis and illustrated in the experimental examples herein may be isolated by standard methods and purification can be achieved by conventional means known to those skilled in the art, such as distillation, recrystallization and chromatography techniques.

The compounds of the present invention which contain one or more double bonds and/or asymmetric centers are capable of existing in various stereoisomeric forms. All such individual forms, and mixtures thereof, are included within the scope of the invention. The various isomers can be obtained by standard methods. For example, cis/trans mixtures can be separated into the individual stereoisomers by stereoselective synthesis, or by separation of the mixtures by fractional crystallization or chromatography techniques.

A number of the compounds of the present invention are capable of forming addition salts with inorganic and organic acids. The pharmaceutically acceptable acid salts of the compounds of the present invention are those which form non-toxic addition salts, such as, but not limited to, the hydrochloride, hydrobromide, hydroiodide, nitrate, sulfate or acetate, fumarate, tartrate, succinate, maleate, glucronate, saccharate, benzoate, methanesulfonate, benzenesulfonate, *p*-toluenesulfonate and pamoate (i.e., 1,1'-methylene-*bis*-(2-hydroxy-3-naphthoate)) salts.

The compounds of the invention which have also acidic groups are capable of forming base salts with various pharmaceutically acceptable cations. Examples of such salts include the alkali metal or alkaline earth metal salts and particularly, the sodium or potassium salts. These salts are all prepared by conventional techniques. For example, these salts can be easily prepared by treating the aforementioned compounds with an aqueous solution containing the desired pharmaceutically acceptable cation, and then evaporating the resulting solution to dryness, preferable under reduce pressure. Alternatively, they may be also be prepared by mixing together with a lower alkoxide, and then evaporating the resulting solution to dryness in the same manners as before. In either case, stoichiometric quantities of reagents are preferably employed in order to ensure completeness of reaction and maximum production of yields of the desired final product.

Also included within the scope of this invention are bioprecursors (also called pro-drugs) of the compounds of the formula (I). A bioprecursor of a compound of the formula (I) is a chemical derivative thereof which is readily converted back into the parent compound of the formula (I) in biological systems. In particular, a bioprecursor of a compound of the formula (I) is converted back to the parent compound of the formula (I) after the bioprecursor has been administered to, and absorbed by, a mammalian subject, e.g., a human subject. For example, it is possible to make a bioprecursor of the compound of the formula (I) in which X¹ is a hydroxy group by making an ester of the hydroxy group. Typical esters are simple alkanoate esters, such as acetate, propionate and butyrate. In addition, when X¹ is a hydroxy group, bioprecursors can be made by converting the hydroxy group to an acyloxymethyl derivative (e.g., a pivaloyloxymethyl derivative) by reaction with an acyloxymethyl halide (e. g., pivaloyloxymethyl chloride). When the compounds of the formula (I) of this invention may form solvates such as hydrates, such solvates are included within the scope of this invention.

### Biological evaluation

The activity of the compounds of the formula (I) of the present invention, is demonstrated by the following assays.

Human umbilical vein endothelial cells (HUVEC), which was characterized by positive staining with von Willibrand's factor and an uptake of acetylated low-density lipoproteins, was purchased from Morinaga Bioscience Lab., Yokohama, Japan. HUVEC was maintained in E-GM UV (from Kurashikibouseki Co., Neyagawa, Japan) in 5% CO₂/95% air at 37 °C. PGE₂, TXB₂ and 6-keto-PGF_{1α} were from Cayman Chemical Co. (Ann Arbor, USA). Recombinant human interleukin-1β (hIL-1β) was from R&D Systems (Minneapolis, USA). RIA kits for PGE₂, TXB₂ and 6-keto-PGF_{1α} were from Amersham (Tokyo, Japan). Indomethacin and other reagents were from Sigma Chemical Co. (St. Louis, USA). Dexamethasone (decadron[Trademark]) was from Banyu Pharmaceutical Co. (Tokyo, Japan). Vacutainer[Trademark] was from Becton Dickinson (Bedford, USA). Male Sprague-Dawley rats were purchased from Charles River (Hino, Japan).

### Human cell-based COX-1 assay

Human cell based COX-1 assay was carried out essentially according to a previously described procedure (Grossman *et al.*, *Inflam. Res.,* **44**, *1995*, 253). Human peripheral blood was obtained from healthy volunteers using Vacutainer[Trademark] containing 1/10 volume of 3.8% sodium citrate solution. After centrifugation, platelet-rich plasma was washed with 0.14 M sodium chloride containing 12 mM Tris-HCl (pH 7.4) and 1.2 mM EDTA. Resulting platelet was washed with 20 mM Hepes-Ca²⁺ free Hanks buffer containing 0.2% BSA. Washed human platelet (WHP) was suspended at the concentration of 2.85 x 10⁷ cells/ml in the above buffer and stored at room temperature until use. Immediately before assay, 10 µl of 12.6 mM CaCl₂ was added to 70 µl WHP suspension (2.0 x 10⁷ cells/ml in a 96-well U bottom plate). Platelets were incubated with a test compound dissolved in DMSO (final concentration less than 0.01%) and A23187 (final 10 µM) at 37 °C for 15 min. Reaction was stopped by addition of EDTA (final 7.7 mM). TXB₂ in the supernatant was quantitated by RIA.

### Human cell-based COX-2 assay

Human cell based COX-2 assay was carried out essentially according to a previously described procedure (Moore *et al., Inflam. Res.,* **45**, *1996,* 54*).* Confluent HUVEC in a 96-well plate were washed with 100 µl of RPMI1640 containing 2% FCS and stimulated with hIL-1β (final 300 U/ml) at 37 °C for 24 hr. HUVEC pretreated with hIL-1β were washed with 20 mM Hepes-Hanks buffer containing 0.2% BSA. The incubation was initiated in 100 µl of the above buffer, A23187 (final 30 µM) and a test compound dissolved in DMSO (final concentration less than 0.01%) at 37 °C for 15 min. 6-Keto-PGF1α, a stable metabolite of PGI₂, in the supernatant was quantitated by RIA.

### Carrageenan induced foot edema in rats

Male Sprague-Dawley rats (5 weeks old), fasted overnight, were injected intradermally λ-carrageenan (0.1 ml of 1% w/v suspension in saline) into right hind paw as previously reported. (Winter *et al*.*, Proc. Soc. Exp. Biol. Med.,* **111**, *1962,* 544; Lombardino *et al*., *Arzneim. Forsch.,* **25**, *1975,* 1629) Foot volume was measured by water displacement using a plethysmometer (Unicom Co., Yachiyo, Japan) before and 3 h after carrageenan injection. Test compounds were suspended in 0.1% w/v methylcellulose and dosed orally in a volume of 2.5 ml per 100 g body weight 1 h before carrageenan injection.

### Measurement of PGE₂ in inflammatory site and stomach in rats

Determination of PGE₂ synthesized in the inflammatory site was carried essentially according to a previously described method (Opas *et al.*, *Biochem. Pharmacol.,* **36**, *1987,* 547). Foot edema in male Sprague-Dawley rats (5 weeks old) was induced by subplanter injection of 0.1 ml of 1% w/v λ-carrageenan suspension. Animals were sacrificed by cervical dislocation 3 h following carrageenan injection. The foot was amputated, frozen in liquid nitrogen and stored at -80 °C until analysis. The stomach of these animals were excised, frozen in liquid nitrogen and stored at -80 °C until analysis. The frozen foot was crushed, mixed with 7 ml of ethanol containing 10 µg/ml of indomethacin, pulverized in a Waring blender and clarified by centrifugation at 3,000 r.p.m. for 10 min at 4 °C. The frozen stomach was mixed with 7 ml of ethanol containing 10 µg/ml of indomethacin, homogenized by Polytrone and clarified by centrifugation at 3,000 r.p.m. for 10 min at 4 °C. PGE₂ was extracted by a Sep-Pak(Trademark) C18 cartridge (from Waters, Milford, USA) and dried in vacuum. Samples were diluted to a final volume of 0.5 ml with assay buffer (PBS containing 0.1% w/v gelatin) and the level of PGE₂ was quantitated by RIA according to Amersham protocol. Test compounds were suspended in 0.1% w/v methylcellulose and dosed 1 h before carrageenan injection. Dexamethasone was dissolved in saline and administered subcutaneously 3 h before carrageenan injection.

### Gastric ulceration in rats

The gastric ulcerogenicity of test compound was assessed using previously described standard method (Ezer *et al*., *J. Pharm. Pharmacol.,* **28,** *1976,* 655). Male Sprague-Dawley rats (5 weeks old), fasted overnight, were used in this assay. Compounds were suspended in 0.1% w/v methylcellulose and dosed orally in a volume of 1.0 ml per 100 g body weight. Six hours after compound administration, the animals were sacrificed by cervical dislocation. The stomach was removed and inflated with 10 ml of 1% formalin solution. The stomach was opened by cutting along the greater curvature and the incidence of ulcer including ecchymosis was evaluated by all-or-none method. Rats refrained from water during experiments. The half-ulcerogenic dose (UD₅₀) value, i.e., dose required to induce at least one gastric lesion or one hemorrhagic erosion in 50% of the animals tested, was calculated by non-linear equation; % Control = 100/(1 + [Dose]/UD₅₀).

### Data Analysis

A statistical program package SYSTAT for Macintosh (SYSTAT, INC.) was used. Differences between compound treated group and control group were tested for using ANOVA. The IC₅₀ or ED₅₀ value was calculated from the equation for the log-linear regression line of concentration (dose) versus percent inhibition.

Most compounds prepared in the Working Examples as described herein after were tested by these methods, and showed IC₅₀ values of 0.01 µM to 1.0 µM with respect to inhibition of COX-2.

COX-2 selectivity can be determined by ratio in terms of IC₅₀ value of COX-1 inhibition to COX-2 inhibition. In general, it can be said that a compound showing a COX-2/COX-1 inhibition ratio of more than 2 has good COX-2 selectivity.

Compounds prepared in Examples 1, 14, 17 and 21 as described below showed COX-2/COX-1 inhibition ratio of more than 10.

The following examples contain detailed descriptions of the methods of the preparation of compounds of formula (I). These detailed descriptions fall within the scope, and serve to exemplify, the above described General Synthetic Procedures which form part of the invention. These detailed descriptions are presented for illustrative purposes only and are not intended as a restriction in the scope of the invention. All parts are by weight and temperatures are in Degrees centigrade unless otherwise noted.

### Examples and Preparations

The present invention is illustrated by the following examples and preparations. However, it should be understood that the invention is not limited to the specific details of these examples and preparations. Melting points were taken with a Buchi micro melting point apparatus and uncorrected. Infrared Ray absorption spectra (IR) were measured by a Shimazu infrared spectrometer (IR-470). ¹H and ¹³C nuclear magnetic resonance spectra (NMR) were measured in CDCl₃ by a JEOL NMR spectrometer (JNM-GX270, 270MHz) unless otherwise indicated and peak positions are expressed in parts per million (ppm) downfield from tetramethylsilane. The peak shapes are denoted as follows: s, singlet; d, doublet; t, triplet; m, multiplet; br, broad.

### Example 1

### (E)-1-Phenyl-2-styryl-1H-benzimidazole

To a stirred solution of *N*-phenyl-*o*-phenylenediamine (1.0 g, 5.4 mmol) in toluene (30 ml) was added cinnamoyl chloride (0.90 g, 5.4 mmol) in small portions and the mixture was heated under reflux overnight. The reaction mixture was cooled and poured into saturated aqueous sodium bicarbonate solution (50 ml) and extracted with ethyl acetate (100 ml). The organic extract was washed consecutively with water (50 ml) and brine (50 ml), dried (sodium sulfate) and concentrated in vacuo. The residue was purified by column chromatography (silica gel, 150 g; n-hexane/ethyl acetate (4/1)) to give pink solids. Recrystallization from diisopropyl ether afforded 1.35 g (84%) of the titled compound as white solids. This compound is used in a pharmaceutical composition.
MW: 296.37
mp: 133.8-134.5 °C
¹H-NMR (CDCl₃) δ: 7.97 (1 H, d, J = 15.8 Hz), 7.84 (1 H, d, J = 8.1 Hz), 7.67 -7.56 (3 H, m), 7.50-7.42 (4 H, m), 7.38-7.16 (6 H, m), 6.85 (1 H, d, J = 15.8 Hz)

### Example 2

### (E)-2-(4-Fluorostyryl)-1-phenyl-1H-benzimidazole

The titled compound was prepared from *N*-phenyl-*o*-phenylenediamine and *p*-fluorocinnamoyl chloride (Gerig, J. T.; McLeod, R. S. *Can. J. Chem.,* 53, 1975, 513) according to the preparation of (*E*)-1-phenyl-2-styryl-1*H*-benzimidazole (example 1).
MW: 314.37
mp: 108.7-109.5 °C
¹H-NMR (CDCl₃) δ: 7.93 (1 H, d, J = 16.1 Hz), 7.83 (1 H, d; J = 8.1 Hz), 7.68-7.56 (3 H, m), 7.49-7.40 (4 H, m), 7.33 (1 H, ddd, J = 7.7, 7.7, 1.8 Hz), 7.27-7.16 (2 H, m), 7.07-6.98 (2 H, m), 6.76 (1 H, d, J = 16.1 Hz)

### Example 3

### (E)-2-(2-Fluorostyryl)-1-phenyl-1H-benzimidazole hydrochloride

The titled compound was prepared from *N*-phenyl-*o*-phenylenediamine and o-fluorocinnamoyl chloride (Gerig, J. T.; McLeod, R. S. Can. J. Chem., 53, 1975, 513) according to the preparation of (*E*)-2-(3-fluorostyryl)-1-phenyl-1*H*-benzimidazole (example 1). The free base was dissolved into methanol, treated with a 10% methanol solution of hydrogen chloride and concentrated to dryness. The residue was recrystallised from diisopropyl ether to give the titled compound as white needles.
MW: 350.83
mp: 225.0-228.0 °C
¹H-NMR (DMSO-d₆) δ: 8.27 (1 H, d, J = 16.5 Hz), 7.91 (1 H, d, J = 8.1 Hz), 7.82-7.69 (6 H, m), 7.63-7.46 (3 H, m), 7.39-7.25 (3 H, m), 7.00 (1 H, d, J = 16.5 Hz)

### Example 4

### (E)-5-Nitro-1-phenyl-2-styryl-1H-benzimidazole

The titled compound was prepared from 5-nitro-2-anilinoaniline (Brand, K.; Wild, E. Chem. Ber., 56, 1923, 105) and cinnamoyl chloride according to the preparation of (*E*)-1-phenyl-2-styryl-1*H*-benzimidazole (example 1).
MW: 341 .37
mp: 173.8-174.7 °C
¹H-NMR (CDCl₃) δ: 8.72 (1 H, d, J = 2.2 Hz), 8.16 (1 H, dd, J = 8.8, 2.2 (Hz), 8.04 (1 H, d, J = 16.1 Hz), 7.72-7.62 (3 H, m), 7.52-7.42 (4 H, m), 7.38-7.31 (3 H, m), 7.22 (1 H, d, J = 8.8 Hz), 6.81 (1 H, d, J = 16.1 Hz)

### Example 5

### (E)-5-Bromo-1-phenyl-2-styryl-1H-benzimidazole

To a stirred solution of (*E*)-5-amino-1-phenyl-2-styryl-1*H*-benzimidazole (0.31 g, 1.0 mmol) in 47% hydrobromic acid (5 ml) and water (2 ml) was added an aqueous (1 ml) solution of sodium nitrite (84 mg, 1.2 mmol) at -5 °C and the reaction mixture was stirred for 10 min at the same temperature. The resultant diazonium salt was added to a solution of copper(I) bromide (0.30 g, 2.0 mmol) in 47% hydrobromic acid (5 ml) cooled to -5 °C and the reaction mixture was stirred for 30 min at 0 °C and for 30 min at room temperature. *N*,*N*,*N'*,*N'*-Tetramethylethylenediamine (5ml) and a 4 *N* aqueous solution of potassium hydroxide (30 ml) were added consecutively to the reaction mixture. The mixture was extracted with ethyl acetate (100 ml) and the ethyl acetate extract was washed consecutively with water (50 ml) and brine (50 ml), dried (sodium sulfate) and concentrated in vacuo. The reside was purified by column chromatography (silica gel, 50 g; *n*-hexane/ethyl acetate (4/1)) to give white solids. Recrystallization from diisopropyl ether gave 61 mg (16%) of the titled compound as white solids.
MW: 375.27
mp: 164.3-165.5 °C
¹H-NMR (CDCl₃) δ: 7.95 (1 H, d, J = 16.1 Hz), 7.95 (1 H, d, J =.1.8 Hz), 7.67-7.56 (3 H, m), 7.49-7.39 (4 H, m), 7.38-7.28 (4 H, m), 7.04 (1 H, d, J = 8.8 Hz), 6.81 (1 H, d, J = 16.1 Hz)

### Example 6

### (E)-5-Cyano-1-phenyl-2-styryl-1H-benzimidazole

A mixture of (*E*)-5-bromo-1-phenyl-2-styryl-1*H*-benzimidazole (1.54 g, 4.1 mmol) and copper(I) cyanide (0.82 g, 8.2 mmol) in 1-methyl-2-pyrroridone (10 ml) was refluxed for 2 h. Then, *N*,*N*,*N'*,*N'*-tetramethylethylenediamine (50ml) was added and the mixture partitioned between ethyl acetate (200 ml) and saturated aqueous sodium bicarbonate solution (200ml). The organic phase was separated and the aqueous phase was extracted with ethyl acetate (200 ml). The combined organic phase was washed with brine (300 ml), dried (sodium sulfate) and concentrated. The residue was purified by column chromatography (silica gel, 150 g; *n*-hexane/ethyl acetate (3/1)) and obtained solids (1.05 g, 80%) recrystallized from ethyl acetate/diisopropyl ether to afford the titled compound as off white solids.
MW: 321.38
mp:191.3-192.4 °C
¹H-NMR (CDCl₃) δ: 8.12 (1 H, d, J = 1.8 Hz), 8.01 (1 H, d, J = 16:1 Hz), 7.71-7.61 (3 H, m), 7.50-7.42 (5 H, m), 7.38-7.11 (3 H, m), 7.23 (1 H, d, J = 8.4 Hz), 6.80 (1 H, d, J = 16.1 Hz)

### Example 7

### (E)-2-(2-Cyclopentylvinyl)-1-phenyl-1H-benzimidazole hydrochloride

To a stirred solution of (*E*)-3-cyclopentylacrylic acid (0.40 g, 2.85 mmol, Roth, R.; Erlenmeyer, H. Helv. Chim. Acta, 38, 1955, 1276) in dichloromethane (20 ml) at 0 °C under a nitrogen atmosphere was added oxalyl chloride (1.46 g, 11.5 mmol). The mixture was stirred at 0 °C for 40 min and then at room temperature for 100 min. Evaporation of volatiles under reduced pressure gave 3-cyclopentylacryloyl chloride as a colorless liquid, which was used without purification.

To a stirred solution of *N*-phenyl-*o*-phenylenediamine (0.38 g, 3.0 mmol) in xylenes (35ml) was added a solution of 3-cyclopentylacryloyl chloride (2.85 mmol) in xylenes (20 ml) during 10 min in a dropwise manner. The resulting mixture was stirred at room temperature for 80 min and then at reflux to remove water using a Dean-Stark apparatus for about 13 h. To the mixture was added *p*-toluenesulfonic acid monohydrate (0.10 g) and the reaction was continued for 10 h. After cooling, volatiles were removed by evaporation and the residue was dissolved into a mixture of ethyl acetate (100 ml) and saturated aqueous sodium bicarbonate solution (100 ml). The organic layer was dried (magnesium sulfate) and concentrated to dryness. Silica-gel column chromatotgraphy (silica gel, 100 g; *n*-hexane/ethyl acetate (4/1 to 7/3)) afforded 0.15 g (18%) of (*E*)-2-(2-cyclopentylvinyl)-1-phenyl-1*H*-benzimidazole as an amber oil. The oil was dissolved into ethyl ether and hydrogen chloride gas was passed through the stirred solution. Volatiles were removed by evaporation and the residue was triturated with diisopropyl ether. Precipitates were collected by suction, washed with diisopropyl ether and dried under vacuum to give the titled compound as white solids.
MW: 324.86
mp: 136.0-137.0 °C (decomposition)
¹H-NMR (DMSO-d₆) δ: 7.86-7.60 (6 H, m), 7.57-7.38 (2 H, m), 7.32-7.11 (2 H, m), 6.23 (1H, d, J = 15.8 Hz), 2.78-2.66 (1H, m), 1.82-1.75 (2 H, m), 1,73-1.50 (4 H, m), 1.45-1.29 (2 H, m)

### Example 8

### (E)-1-Phenyl-2-[2-(2-pyridyl)vinyl]-1H-benzimidazole

### 1. N-(2-Anilinophenyl)-3-(2-pyridyl)acrylamide

To a stirred solution of (*E*)-3-(2-pyridyl)acrylic acid (0.44 g, 3.0 mmol, Ried, W.; Keller, H. Chem. Ber., 89, 1955, 2578), *N*-phenyl-*o*-phenylenediamine (0.61 g, 3.3 mmol) and 1-(3-dimethylaminopropyl)-3-ethylcarbodiimide hydrochloride (0.69 g, 3.6 mmol) in dimethylformamide (5 ml) at -20 °C under a nitrogen atmosphere was added triethylamine (0.7 ml). The resulting mixture was stirred at -18 to -8 °C for 40 min and then at room temperature for 8 h. The mixture was diluted with water (50 ml) and extracted with ethyl acetate (50 ml). The organic extract was washed consecutively with water (50 ml) and brine (50 ml), dried (magnesium sulfate) and concentrated to dryness. Column chromatography (silica gel, 85 g; *n*-hexane/ethyl acetate (1/1)) afforded 0.40 g (42%) of *N*-(2-anilinophenyl)-3-(2-pyridyl)acrylamide as an orange liquid.

¹H-NMR (CDCl₃) δ: 8.63-8.59 (1 H, m), 8.24-8.16 (1 H, m), 8.03 (1 H, br. s), 7.75-7.68 (1 H, m), 7.69 (1 H, d, J = 15.0 Hz), 7.40-7.36 (1 H, m), 7.30-7.12 (6 H, m), 7.08 (1 H, d, J = 15.0 Hz), 6.91-6.85 (1 H, m), 6.82-6.75 (2 H, m), 5.55 (1 H, br. s).

### 2.(E)-1-Phenyl-2-[2-(2-pyridyl)vinyl]-1H-benzimidazole

A stirred mixture of *N*-(2-anilinophenyl)-3-(2-pyridyl)acrylamide, *p*-toluenesulfonic acid monohydrate (0.48 g, 2.5 mmol) and xylenes (80 ml) was heated to reflux to remove water using a Dean-Stark apparatus for 4 h. After cooling, the mixture was concentrated to dryness and the residue shaken with ethyl acetate (100 ml) and saturated aqueous sodium bicarbonate solution (50 ml). The aqueous layer was separated and extracted with ethyl acetate (2 x 100 ml). The combined organic layers were dried (magnesium sulfate) and concentrated to dryness. The crude product was purified by column chromatography (silica gel, 85 g; *n*-hexane/ethyl acetate (1/2)) to afford 0.29 g of product as reddish solids. Recrystallization from diisopropyl ether/ethyl acetate (5/1) afforded 0.17 g (45%) of the titled compound as white solids.
MW: 297.36
mp: 144.0-145.0 °C
¹H-NMR (CDCl₃) δ: 8.58-8.53 (1 H, m), 7.99 (1 H, d, J = 15.6 Hz), 7.88-7.83 (1 H, m), 7.71-7.57 (4H, m), 7.52 (1 H, d, J = 15.6 Hz), 7.49-7.44 (2 H, m), 7.38-7.14 (5 H, m)

### Example 9

### (E)-1-Phenyl-2-[2-(4-pyridyl)vinyl]-1H-benzimidazole

The titled compound was prepared from *N*-phenyl-*o*-phenylenediamine and (*E*)-3-(4-pyridyl)acrylic acid according to the preparation of (*E*)-1-phenyl-2-[2-(2-pyridyl)vinyl]-1*H*-benzimidazole (example 8).
MW: 297.36
mp: 116.0-117.5 °C
¹H-NMR (CDCl₃) δ: 8.60-8.56 (2 H, m), 7.88-7.83 (1 H, m), 7.87 (1 H, d, J = 16.1 Hz), 7.70-7.56 (3H, m), 7.48-7.42 (2 H, m), 7.39-7.32 (1 H, m), 7.31-7.19 (4 H, m), 7.04 (1 H, d, J = 16.1 Hz)

### Example 10

### (E)-2-[2-(2-Oxazolyl)vinyl]-1-phenyl-1H-benzimidazole

### 1. Ethyl (E)-2-(1-phenyl-1H-benzimidazol-2-yl)propenoate

Ethyl (*E*)-2-(1-phenyl-1*H*-benzimidazol-2-yl)propenoate was prepared from *N*-phenyl-*o*-phenylenediamine and ethyl 3-chlorocarbonylacrylate (Home, S.; Taylor, N.; Collins, S.; Rodrigo, R. J. Chem. Soc. Perkin Trans. *I*, 12, 1991, 3047) according to the preparation of (*E*)-1-phenyl-2-styryl-1*H*-benzimidazole (example 1).
¹H-NMR (CDCl₃) δ: 7.88-7.83 (1 H, m), 7.66-7.55 (3 H, m), 7.42 (1 H, d, J = 15.4 Hz), 7.41-7.19 (5 H, m), 7.15 (1 H, d, J = 15.4 Hz), 4.23 (2 H, q, J = 7.0 Hz), 1.30 (3 H, t, J = 7.0 Hz).

### 2. (E)-2-(1-Phenyl-1H-benzimidazol-2-yl)pronenoic acid

To a solution of ethyl (*E*)-2-(1-phenyl-1*H*-benzimidazol-2-yl)propenoate (3.74 g, 12.8 mmol) in a mixture of methanol (18 ml) and tetrahydrofuran (18 ml) was added a 4 N aqueous solution of lithium hydroxide (6 ml, 24 mmol) and the reaction mixture was stirred for 3 h at room temperature. To the reaction mixture was added 4 N hydrochloric acid (6 ml, 24 mmol) at 0 °C. The precipitated solids were collected by the filtration and dried under reduced pressure to afford 2.70 g (80%) of (*E*)-2-(1-Phenyl-1*H*-benzimidazol-2-yl)propenoic acid as white solids.
¹H-NMR (DMSO-d₆) δ: 7.89 (1 H, d, J = 7.3 Hz), 7.84-7.62 (5 H, m), 7.48-7.26 (4 H, m), 6.97 (1 H, d, J = 15.4 Hz).

### 3. 3-(1-Phenyl-1H-benzimidazol-2-yl)acrylamide

To a stirred solution of 3-(1-phenyl-1*H*-benzimidazol-2-yl)acrylic acid (1.06 g, 4 mmol) in dichloromethane (80 ml) at 0 °C under a nitrogen atmosphere was added oxalyl chloride (3.05 g, 24 mmol) and the resulting suspension was stirred at 0 °C for 20 min and then at room temperature for 2.5 h. Volatiles were removed by evaporation and the residue was added in portions to a stirred 25% aqueous solution of ammonia (40 ml). After 130 min, solids were collected by suction, washed with water and dried under vacuum at 50 °C to give 1.04 g (98%) of the titled compound as off-white solids.
¹H-NMR (CDCl₃) δ: 7.86-7.91 (1 H, m), 7.66-7.55 (3 H, m), 7.45 (1 H, d, J = 15.0 Hz), 7.43-7.19 (6 H, m), 5.65 (1 H, br.s), 5.55 (1 H, br.s).

### 4. (E)-2-[2-Oxazolyl)vinyl]-1-phenyl-1H-benzimidazole

A mixture of 3-(1-phenyl-1*H*-benzimidazol-2-yl)acrylamide (0.62 g, 2.35 mmol), vinylene carbonate (0.26 g, 3.0 mmol) and polyphosphoric acid (6.2 g) was stirred at 170 °C. After 2 h, ice was added and the mixture was basified with a 10% aqueous solution of potassium hydroxide (100 ml) and extracted with dichloromethane/isopropyl alcohol (9/1, 150 ml + 50 ml). The combined organic layers were washed consecutively with water (2 x 100 ml) and brine (100 ml), dried (magnesium sulfate) and concentrated to dryness. Purification by column chromatography (silica gel 100 g, ethyl acetate) afforded 66 mg of product as pale green solids which were recrystallized from diisopropyl ether to afford 36 mg (5%) of the titled compound as tan solids.
MW: 287.32
mp: 155.5-156.0 °C
¹H-NMR (CDCl₃) δ: 7.89-7.84 (1 H, m), 7.73 (1 H, d, J = 16.1 Hz), 7.68-7.54 (4 H, m), 7.47-7.42 (2 H, m), 7.39-7.32 (1 H, m), 7.31-7.25 (1 H, m), 7.27 (1 H, d, J = 16.1 Hz), 7.23-7.18 (2 H, m)

The chemical structures of the compounds prepared in the Examples 1 to 10 are summarized in the following table.

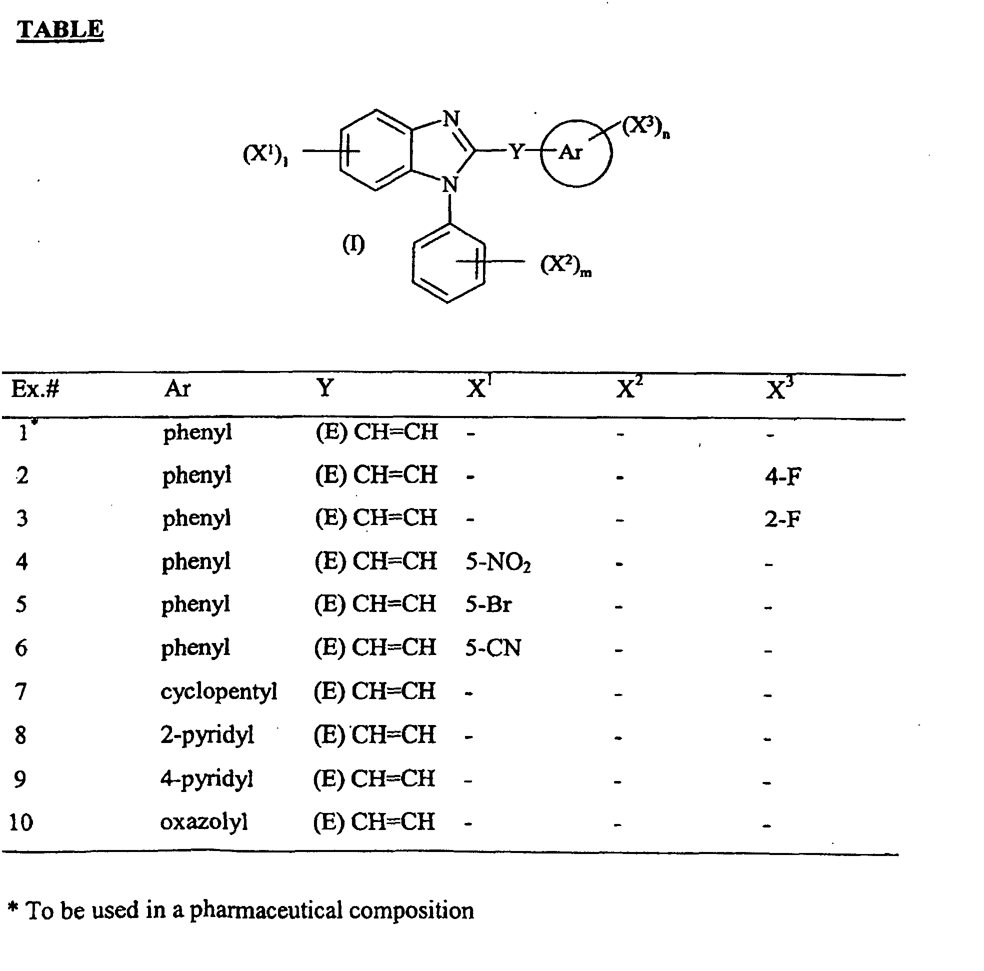

## Claims

1. A compound of the following formula: 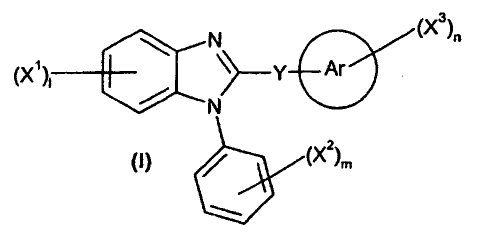 and the pharmaceutically acceptable salts thereof wherein
Ar is phenyl, C₃₋₈ cycloalkyl or heteroaryl which is connected to Y through a carbon atom, the heteroaryl being selected from pyridyl and oxazolyl;
X¹ is H, halo, amino, cyano or nitro;
X² and X³ are halo;
Y is -CR¹=CR²-, wherein R¹ and R² are independently H or methyl;
1 is 0 or 1;
m and n are independently 0 or 1;
with the proviso that when Ar is phenyl; and l, m and n are 0, Y is not -CH=CH-

2. A compound according to claim 1, wherein Ar is phenyl or C₃₋₈ cycloalkyl; X² and X³ are halo; X¹ is H, halo, amino, cyano or nitro, which is attached to 5- or 6-ring position of the benzimidazole ring system; and Y is -CR¹=CR²-, wherein R¹ and R² are independently H or methyl.

3. A compound according to claim 2, wherein Ar is phenyl, cyclopentyl or cyclohexyl; X² and X³ are fluoro; X¹ is H, fluoro, chloro, bromo, amino, cyano or nitro, which is attached to 5-ring position of the benzimidazole ring system; and Y is -CH=CH-.

4. A compound according to claim 1 selected from
(*E*)-2-(4-fluorostyryl)-1-phenyl-1H-benzimidazole;
(*E*)-2-(2-fluorostyryl)-1-phenyl-1H-benzimidazole hydrochloride;
(*E*)-5-nitro-1-phenyl-2-styryl-1H-benzimidazole;
(*E*)-5-amino-1-phenyl-2-styryl-1H-benzimidazole dihydrochloride;
(*E*)-5-bromo-1-phenyl-2-styryl-1H-benzimidazole;
(*E*)-5-cyano-1-phenyl-2-styryl-1H-benzimidazole;
(*E*)-2-(2-cyclohexylvinyl)-1-phenyl-1H-benzimidazole;
(*E*)-2-(2-cyclopentylvinyl)-1-phenyl-1H-benzimidazole hydrochloride; and
*(E)-*1-phenyl-2-[2-(2-pyridyl)vinyl]-1H-benzimidazole.

5. A compound according to claim 4 selected from
(*E*)-5-nitro-1-phenyl-2-styryl-1H-benzimidazole;
(*E*)-5-cyano-1-phenyl-2-styryl-1H-benzimidazole; and
(*E*)-2-(2-cyclopentylvinyl)-1-phenyl-1H-benzimidazole hydrochloride.

6. A pharmaceutical composition which comprises a compound of the following formula (I): 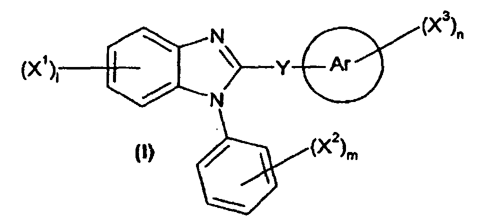 and the pharmaceutically acceptable salts thereof wherein
Ar is phenyl, C₃₋₈ cycloalkyl or heteroaryl which is connected to Y through a carbon atom, the heteroaryl being selected from pyridyl and oxazolyl;
X¹ is H, halo, amino, cyano or nitro;
X² and X³ are halo;
Y is -CR¹=CR²-, wherein R¹ and R² are independently H or methyl;
1 is 0 or 1;
m and n are independently 0 or 1;
and a pharmaceutically inert carrier or diluent.

7. A pharmaceutical composition according to claim 6, wherein Ar is phenyl or C₃₋₈ cycloalkyl; X² and X³ are halo; X¹ is H, halo, amino, cyano or nitro, which is attached to 5- or 6-ring position of the benzimidazole ring system; and Y is -CR¹=CR²-, wherein R¹ and R² are independently H or methyl.

8. A pharmaceutical composition according to claim 7, wherein Ar is phenyl, cyclopentyl or cyclohexyl; X² and X³ are fluoro; X1 is H, fluoro, chloro, bromo, amino, cyano or nitro, which is attached to 5-ring position of the benzimidazole ring system; and Y is -CH=CH-.

9. A pharmaceutical composition according to claim 6, wherein the compound is selected from
(*E*)-1-phenyl-2-styryl-1H-benzimidazole;
(*E*)-2-(4-fluorostyryl)-1-phenyl-1H-benzimidazole;
(*E*)-2-(2-fluorostyryl)-1-phenyl-1H-benzimidazole hydrochloride;
(*E*)-5-nitro-1-phenyl-2-styryl-1H-benzimidazole;
(*E*)-5-amino-1-phenyl-2-styryl-1H-benzimidazole dihydrochloride;
(*E*)-5-bromo-1-phenyl-2-styryl-1H-benzimidazole;
(*E*)-5-cyano-1-phenyl-2-styryl-1H-benzimidazole;
(*E*)-2-(2-cyclohexylvinyl)-1-phenyl-1H-benzimidazole;
(*E*)-2-(2-cyclopentylvinyl)-1-phenyl-1H-benzimidazole hydrochloride; and
(*E*)-1-phenyl-2-[2-(2-pyridyl)vinyl]-1H-benzimidazole.

10. A pharmaceutical composition according to claim 9, wherein the compound is selected from
(*E*)-1-phenyl-2-styryl-1H-benzimidazole;
(*E*)-5-nitro-1-phenyl-2-styryl-1H-benzimidazole;
(*E*)-5-cyano-1-phenyl-2-styryl-1H-benzimidazole; and
(*E*)-2-(2-cyclopentylvinyl)-1-phenyl-1H-benzimidazole hydrochloride.

11. A compound of the formula (I) or a pharmaceutically acceptable salt or composition thereof as claimed in any one of claims 1 to 5 and 6 to 10, respectively, for use as a medicament.

12. The use of a compound of the formula (I), or of a phamaceutically acceptable salt or composition thereof, as claimed in any one of claims 1 to 5 and 7 to 10, respectively, for the manufacture of a medicament for treating a medical condition in which prostaglandins are implicated as pathogens in a mammalian subject.

## Patentansprüche

1. Verbindung der folgenden Formel 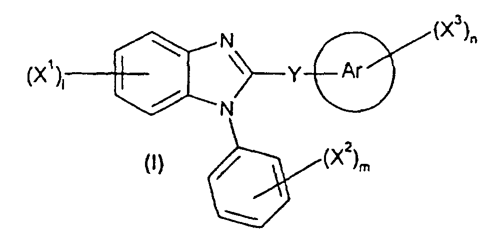 und die pharmazeutisch akzeptablen Salze derselben, worin Ar Phenyl, C₃₋₈-Cycloalkyl oder ein Heteroaryl, das an Y über ein Kohlenstoffatom gebunden ist, wobei das Heteroaryl aus Pyridyl und Oxazolyl ausgewählt ist, bedeutet;
X¹ H, Halogen, Amino, Cyano oder Nitro bedeutet;
X² und X³ Halogen bedeuten;
Y -CR¹=CR²-, wobei R¹ und R² unabhängig voneinander H oder Methyl sind, bedeutet;
l 0 oder 1 ist;
m und n unabhängig voneinander 0 oder 1 sind;
wobei, wenn Ar Phenyl ist, und l, m und n 0 sind, Y nicht -CH=CH- ist.

2. Verbindung nach Anspruch 1, worin Ar Phenyl oder C₃₋₈-Cycloalkyl bedeutet; X² und X³ Halogen bedeuten; X¹ H, Halogen, Amino, Cyano oder Nitro, das an die 5- oder 6-Ringposition des Benzimidazolringsystems gebunden ist, bedeutet; und Y -CR¹=CR²-, wobei R¹ und R² unabhängig voneinander H oder Methyl sind, bedeutet.

3. Verbindung nach Anspruch 2, worin Ar Phenyl, Cyclopentyl oder Cyclohexyl bedeutet; X² und X³ Fluor bedeuten; X¹ H, Fluor, Chlor, Brom, Amino, Cyano oder Nitro, das an die 5-Ringposition des Benzimidazolringsystems gebunden ist, bedeutet; und Y -CH=CH- bedeutet.

4. Verbindung nach Anspruch 1, die ausgewählt ist aus
(E)-2-(4-Fluorstyryl)-1-phenyl-1H-benzimidazol;
(E)-2-(2-Fluorstyryl)-1-phenyl-1H-benzimidazolhydrochlorid;
(E)-5-Nitro-1-phenyl-2-styryl-1H-benzimidazol;
(E)-5-Amino-1-phenyl-2-styryl-1H-benzimidazoldihydrochlorid;
(E)-5-Brom-1-phenyl-2-styryl-1H-benzimidazol;
(E)-5-Cyano-1-phenyl-2-styryl-1H-benzimidazol;
(E)-2-(2-Cyclohexylvinyl)-1-phenyl-1H-benzimidazol;
(E)-2-(2-Cyclopentylvinyl)-1-phenyl-1H-benzimidazolhydrochlorid; und
(E)-1-Phenyl-2-[2-(2-pyridyl)vinyl]-1H-benzimidazol.

5. Verbindung nach Anspruch 4, die ausgewählt ist aus
(E)-5-Nitro-1-phenyl-2-styryl-1H-benzimidazol;
(E)-5-Cyano-1-phenyl-2-styryl-1H-benzimidazol; und
(E)-2-(2-Cyclopentylvinyl)-1-phenyl-1H-benzimidazolhydrochlorid.

6. Pharmazeutische Zusammensetzung, die eine Verbindung der folgenden Formel (I): 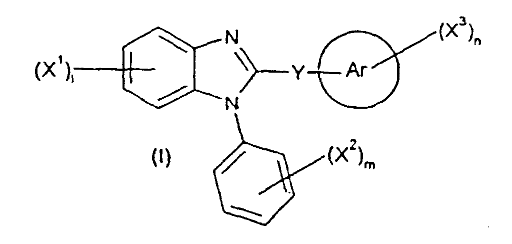 und die pharmazeutisch akzeptablen Salze derselben, worin Ar Phenyl, C₃₋₈-Cycloalkyl oder ein Heteroaryl, das an Y über ein Kohlenstoffatom gebunden ist, wobei das Heteroaryl aus Pyridyl und Oxazolyl ausgewählt ist, bedeutet;
X¹ H, Halogen, Amino, Cyano oder Nitro bedeutet;
X² und X³ Halogen bedeuten;
Y -CR¹=CR²-, wobei R¹ und R² unabhängig voneinander H oder Methyl sind, bedeutet;
l 0 oder 1 ist;
m und n unabhängig voneinander 0 oder 1 sind;
und einen pharmazeutisch inerten Träger oder ein pharmazeutisch inertes Verdünnungsmittel umfasst.

7. Pharmazeutische Zusammensetzung nach Anspruch 6, worin Ar Phenyl oder C₃₋₈-Cycloalkyl bedeutet; X² und X³ Halogen bedeuten; X¹ H, Halogen, Amino, Cyano oder Nitro, das an die 5- oder 6-Ringposition des Benzimidazolringsystems gebunden ist, bedeutet; und Y -CR¹=CR²-, wobei R¹ und R² unabhängig voneinander H oder Methyl sind, bedeutet.

8. Pharmazeutische Zusammensetzung nach Anspruch 7, worin Ar Phenyl, Cyclopentyl oder Cyclohexyl bedeutet; X² und X³ Fluor bedeuten; X¹ H, Fluor, Chlor, Brom, Amino, Cyano oder Nitro, das an die 5-Ringposition des Benzimidazolringsystems gebunden ist, bedeutet; und Y -CH=CH- bedeutet.

9. Pharmazeutische Zusammensetzung nach Anspruch 6, wobei die Verbindung ausgewählt ist aus
(E)-1-Phenyl-2-styryl-1H-benzimidazol;
(E)-2-(4-Fluorstyryl)-1-phenyl-1H-benzimidazol;
(E)-2-(2-Fluorstyryl)-1-phenyl-1H-benzimidazolhydrochlorid;
(E)-5-Nitro-1-phenyl-2-styryl-1H-benzimidazol;
(E)-5-Amino-1-phenyl-2-styryl-1H-benzimidazoldihydrochlorid;
(E)-5-Brom-1-phenyl-2-styryl-1H-benzimidazol;
(E)-5-Cyano-1-phenyl-2-styryl-1H-benzimidazol;
(E)-2-(2-Cyclohexylvinyl)-1-phenyl-1H-benzimidazol;
(E)-2-(2-Cyclopentylvinyl)-1-phenyl-1H-benzimidazolhydrochlorid; und
(E)-1-Phenyl-2-[2-(2-pyridyl)vinyl]-1H-benzimidazol.

10. Pharmazeutische Zusammensetzung nach Anspruch 9, wobei die Verbindung ausgewählt ist aus
(E)-1-Phenyl-2-styryl-1H-benzimidazol;
(E)-5-Nitro-1-phenyl-2-styryl-1H-benzimidazol;
(E)-5-Cyano-1-phenyl-2-styryl-1H-benzimidazol; und
(E)-2-(2-Cyclopentylvinyl)-1-phenyl-1H-benzimidazolhydrochlorid.

11. Verbindung der Formel I oder ein pharmazeutisch akzeptables Salz oder eine pharmazeutisch akzeptable Zusammensetzungen derselben gemäß einem der Ansprüche 1 bis 5 bzw. 6 bis 10 zur Verwendung als Medikament.

12. Verwendung einer Verbindung der Formel I oder eines pharmazeutisch akzeptablen Salzes oder einer pharmazeutisch akzeptablen Zusammensetzung derselben gemäß einem der Ansprüche 1 bis 5 bzw. 7 bis 10 zur Herstellung eines Medikaments zur Behandlung eines medizinischen Zustands, an dem Prostaglandine als Pathogene beteiligt sind, bei einem Säugetierobjekt.

## Revendications

1. Composé répondant à la formule suivante : 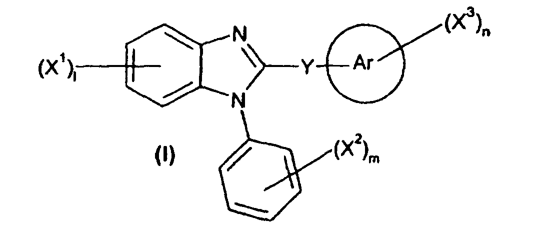 et ses sels pharmaceutiquement acceptables, formule dans laquelle
Ar représente un groupe phényle, cycloalkyle en C₃ à C₈ ou hétéroaryle qui est connecté à Y par un atome de carbone ; le groupe hétéroaryle étant choisi entre les groupes pyridyle et oxazolyle ;
X¹ représente H, un groupe halogéno, amino, cyano ou nitro ;
X² et X³ représentent des groupes halogène ;
Y représente un groupe -CR¹=CR²-, dans lequel R¹ et R² représentent indépendamment H ou un groupe méthyle ;
l est égal à 0 ou 1 ;
m et n sont égaux indépendamment à 0 ou 1 ;
sous réserve que, lorsque Ar représente un groupe phényle et 1, m et n sont égaux à 0, Y ne représente pas un groupe -CH=CH-.

2. Composé suivant la revendication 1, dans lequel Ar représente un groupe phényle ou cycloalkyle en C₃ à C₈ ; X² et X³ représentent des groupes halogéno ; X¹ représente H, un groupe halogéno, amino, cyano ou nitro, qui est fixé à la position 5 ou 6 de noyau du système cyclique benzimidazole ; et Y représente un groupe -CR¹=CR²-, dans lequel R¹ et R² représentent indépendamment H ou des groupes méthyle.

3. Composé suivant la revendication 2, dans lequel Ar représente un groupe phényle, cyclopentyle ou cyclohexyle ; X² et X³ représentent des groupes f luoro ; X¹ représente H, un groupe fluoro, chloro, bromo, amino, cyano ou nitro, qui est fixé à la position 5 de noyau du système cyclique benzimidazole ; et Y représente un groupe -CH=CH-.

4. Composé suivant la revendication 1, choisi entre les suivants :
(*E*)-2-(4-fluorostyryl)-1-phényl-1H-benzimidazole ;
chlorhydrate de (*E*)-2-(2-fluorostyryl)-1-phényl-1H-benzimidazole ;
(*E*)-5-nitro-1-phényl-2-styryl-1H-benzimidazole ;
dichlorhydrate de (*E*)-5-amino-1-phényl-2-styryl-1H-benzimidazole ;
(*E*)-5-bromo-1-phényl-2-styryl-1H-benzimidazole ;
(*E*)-5-cyano-1-phényl-2-styryl-1H-benzimidazole ;
(*E*)-2-(2-cyclohexylvinyl)-1-phényl-1H-benzimidazole ;
chlorhydrate de (*E*)-2-(2-cyclopentylvinyl)-1-phényl-1H-benzimidazole ; et
(*E*)-1-phényl-2-[2-(2-pyridyl)vinyl]-1H-benzimidazole ;

5. Composé suivant la revendication 4, choisi entre les suivants :
(*E*)-5-nitro-1-phényl-2-styryl-1H-benzimidazole ;
(*E*)-5-cyano-1-phényl-2-styryl-1H-benzimidazole ; et
chlorhydrate de (*E*)-2-(2-cyclopentylvinyl)-1-phényl-1H-benzimidazole.

6. Composition pharmaceutique qui comprend un composé répondant à la formule (I) suivante : 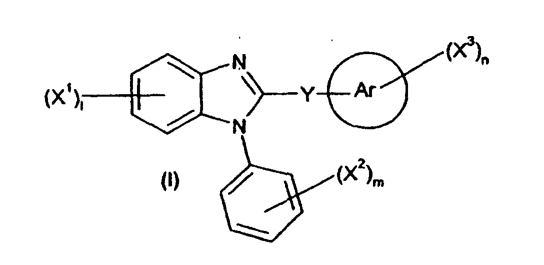 ou un de ses sels pharmaceutiquement acceptables, formule dans laquelle
Ar représente un groupe phényle, cycloalkyle en C₃ à C₈ ou hétéroaryle qui est connecté à Y par un atome de carbone, le groupe hétéroaryle étant choisi entre les groupes pyridyle ou oxazolyle ;
X¹ représente H, un groupe halogéno, amino, cyano ou nitro ;
X² et X³ représentent des groupes halogéno ;
Y représente un groupe -CR¹=CR²-, dans lequel R¹ et R² représentent indépendamment H ou un groupe méthyle ;
l est égal à 0 ou 1 ;
m et n sont égaux indépendamment à 0 ou 1 ;
et un support ou diluant pharmaceutiquement inerte.

7. Composition pharmaceutique suivant la revendication 6, dans laquelle Ar représente un groupe phényle ou cycloalkyle en C₃ à C₈ ; X² et X³ représentent des groupes halogéno ; X¹ représente H, un groupe halogéno, amino, cyano, ou nitro, qui est fixé à la position 5 ou 6 de noyau du système cyclique benzimidazole ; et Y représente un groupe -CR¹=CR²-, dans lequel R¹ et R² représentent indépendamment H ou un groupe méthyle.

8. Composition pharmaceutique suivant la revendication 7, dans laquelle Ar représente un groupe phényle, cyclopentyle ou cyclohexyle ; X² et X³ représentent des groupes fluoro ; X¹ représente H, un groupe fluoro, chloro, bromo, amino, cyano ou nitro, qui est fixé à la position 5 de noyau du système cyclique benzimidazole ; et Y représente un groupe -CH=CH.

9. Composition pharmaceutique suivant la revendication 6, dans laquelle le composé est choisi entre les suivants :
(*E*)-1-phényl-2-styryl-1H-benzimidazole ;
(*E*)-2-(4-fluorostyryl)-1-phényl-1H-benzimidazole ;
dichlorhydrate de (*E*)-2-(2-flurostyryl)-1-phényl-1H-benzimidazole ;
(*E*)-5-nitro-1-phényl-2-styryl-1H-benzimidazole ;
dichlorhydrate de (*E*)-5-amino-1-phényl-2-styryl-1H-benzimidazole ;
(*E*)-5-bromo-1-phényl-2-styryl-1H-benzimidazole ;
(*E*)-5-cyano-1-phényl-2-styryl-1H-benzimidazole ;
(*E*)-2-(2-cyclohexylvinyl)-1-phényl-1H-benzimidazole ;
chlorhydrate de (*E*)-2-(2-cyclopentylvinyl)-1-phényl-1H-benzimidazole ; et
(*E*)-1-phényl-2-[2-(2-pyridyl)vinyl]-1H-benzimidazole ;

10. Composition pharmaceutique suivant la revendication 9, dans laquelle le composé est choisi entre les suivants :
(*E*)-1-phényl-2-styryl-1H-benzimidazole ;
(*E*)-5-nitro-1-phényl-2-styryl-1H-benzimidazole ;
(*E*)-5-cyano-1-phényl-2-styryl-1H-benzimidazole ; et
chlorhydrate de (*E*)-2-(2-cyclopentylvinyl)-1-phényl-1H-benzimidazole.

11. Composé selon la formule (I) ou un de ses sels pharmaceutiquement acceptables ou composition le contenant, suivant l'une quelconque des revendications 1 à 5 et 6 à 10, respectivement, pour l'utilisation en tant que médicament.

12. Utilisation d'un composé de formule (I) ou un de ses sels pharmaceutiquement acceptables ou composition le contenant selon l'une quelconque des revendications 1 à 5 et 7 à 10, respectivement, pour la production d'un médicament destiné au traitement d'états médicaux dans lesquels des postaglandines sont impliquées en tant qu'agents pathogènes chez un sujet consistant en un mammifère.
